# EUROPEAN PATENT SPECIFICATION

(11) **EP 2 745 784 B1**
(45) Date of publication and mention of the grant of the patent: **12.02.2020**
(21) Application number: 13197958.5
(22) Date of filing: 18.12.2013
(51) Int. Cl.: A61B 17/072

(54) **Buttress attachment to the cartridge surface**
Versteifungsbefestigung an der Oberfläche einer Kartusche
Contrefort de fixation sur la surface d'une cartouche

(30) Priority: 19.12.2012 US 201213719630
(43) Date of publication of application: 25.06.2014
(73) Proprietor: Covidien LP, Mansfield, MA 02048 (US)
(72) Inventor: Hodgkinson, Gerald N., Guilford, CT 06437 (US)
(74) Representative: Soames, Candida Jane

(56) References cited:
- EP-A1- 1 256 318
- EP-A1- 2 586 380
- US-A1- 2003 120 284
- US-A1- 2006 173 470

## Description

### BACKGROUND

### 1. Technical field

The present disclosure relates to surgical stapling apparatus including surgical buttresses which can be releasably attached to the surgical stapling apparatus, and in particular, to surgical stapling apparatus having adhesive member disposed on a tissue contacting surface of the surgical stapling apparatus to join the surgical buttresses thereto.

### 2. Background of Related Art

Surgical stapling apparatus are employed by surgeons to sequentially or simultaneously apply one or more rows of fasteners, e.g., staples or two-part fasteners, to body tissue for the purpose of joining segments of body tissue together. Such apparatus generally include a pair of jaws or finger-like structures between which the body tissue to be joined is placed. When the stapling apparatus is actuated, or "fired", longitudinally moving firing bars contact staple drive members in one of the jaws. The staple drive members push the surgical staples through the body tissue and into an anvil in the opposite jaw which forms the staples. If tissue is to be removed or separated, a knife blade can be provided in the jaws of the apparatus to cut the tissue between the lines of staples.

A number of surgical stapling apparatus rely on a knife blade cutting off some portion of the surgical buttress to affect release. These methods typically employ a secondary material or mounting structure in addition to the surgical buttress (e.g., sutures) to provide attachment of the surgical buttress to the surgical stapling apparatus. Typically, firing forces are increased with each material that must be transected by the knife blade in order to release the surgical buttress.

It would be desirable to provide a surgical buttress that may be releasably secured to a surgical stapling apparatus without the need for a secondary material or mounting structure, and without the need for a knife blade to cut the buttress and/or mounting structure to release the surgical buttress from the surgical stapling apparatus, thereby resulting in the use of fewer materials and lower firing forces.

A surgical buttress adhered to a tissue contacting surface by a continuous layer of adhesive extending across the whole width of the buttress is known from US 2006/173470

### SUMMARY

The present invention is defined by the features of the independent claim. Preferred embodiments are given in the dependent claims.

According to one aspect of the present disclosure, an end effector assembly for use with a surgical stapler wherein the end effector comprises a staple cartridge having a tissue contacting surface defining a central longitudinal slot and an anvil plate having a tissue contacting surface defining a central longitudinal slot. A surgical buttress is releasbly disposed on the tissue contacting surfaces of each of the staple cartridge and anvil plate. An adhesive tape is disposed over the central longitudinal slot of each of the staple cartridge and anvil plate and configured retain the respective surgical buttress atop the respective tissue contacting surface. Preferably, the surgical buttress is laser welded to the adhesive tape.

The adhesive tape is secured to the tissue contacting surfaces of each of the staple cartridge and anvil plate surrounding the edges of the respective central longitudinal slots. Additionally, the adhesive tape extends longitudinally between a proximal end and a distal end of the tissue contacting surfaces of each of the staple cartridge and anvil plate.

According to another aspect of the present disclosure, a staple cartridge for use with a surgical stapling apparatus wherein the staple cartridge comprises a cartridge body including a tissue contacting surface defining a plurality of staple retaining slots and having a central longitudinal slot and a staple disposed within each staple retaining slot of the cartridge body. A surgical buttress is releasbly disposed on the tissue contacting surfaces of the cartridge body. An adhesive tape is disposed over the central longitudinal slot of the cartridge body and configured to retain the surgical buttress atop the tissue contacting surface of the cartridge body. Preferably, the surgical buttress is laser welded to the adhesive tape.

The adhesive tape is secured to the tissue contacting surface of the cartridge body surrounding the edges of the central longitudinal slot. Additionally, the adhesive tape extends longitudinally between a proximal end and a distal end of the tissue contacting surface of the cartridge body.

According to another aspect of the present disclosure, a surgical stapling apparatus wherein the surgical stapling apparatus comprises a housing and an end effector being secured to the housing having a staple cartridge assembly having a tissue contacting surface and an anvil assembly having a tissue contacting surface, each of the staple cartridge assembly and anvil assembly having a central longitudinal slot. A surgical buttress is releasbly disposed on the tissue contacting surfaces of each of the staple cartridge assembly and anvil assembly. An adhesive tape is disposed over the central longitudinal slot of each of the staple cartridge assembly and anvil assembly and configured retain the respective surgical buttress atop the respective tissue contacting surface. Preferably, the surgical buttress is laser welded to the adhesive tape.

### BRIEF DESCRIPTION OF THE DRAWINGS

Various embodiments of the presently disclosed interlocking buttress retention systems are disclosed herein with reference to the drawings, wherein:
FIG. 1 is a perspective view of a surgical stapling apparatus according to an embodiment of the present disclosure;
FIG. 2 is a perspective view, with parts separated, of a staple cartridge assembly of the surgical stapling apparatus of FIG. 1, illustrating a surgical buttress associated therewith;
FIG. 3 is a perspective view, with parts separated, of an anvil assembly of the surgical stapling apparatus of FIG. 1, illustrating a surgical buttress associated therewith;
FIG. 4A is a perspective view of the staple cartridge assembly, illustrating the surgical buttress affixed to a staple cartridge;
FIG. 4B is a cross-sectional view taken along line 4B-4B of FIG. 4A;
FIG. 5 is a perspective view of the anvil assembly, illustrating the surgical buttress affixed to an anvil plate;
FIG. 6 is a perspective view of a distal end of the surgical stapling apparatus of FIG. 1, shown in use positioned about a tissue section;
FIG. 7 is a cross-sectional view taken along line 7-7 of FIG. 6;
FIG. 8 is a perspective view of the stapled and divided tissue section of FIG. 6;
FIG. 9A is a perspective view of an illustrative embodiment of a surgical stapling apparatus in accordance with another embodiment of the present disclosure;
FIG. 9B is a side elevational view, partially broken away, of the surgical stapling apparatus of FIG. 9A;
FIG. 10A is a perspective view of an illustrative embodiment of the staple cartridge assembly of the surgical stapling apparatus of FIG. 9A including a surgical buttress in accordance with an embodiment of the present disclosure;
FIG. 10B is a top plan view of the staple cartridge assembly and surgical buttress illustrated in FIG. 10A;
FIG. 11 is perspective view of an intestinal area of a patient, illustrating a method of positioning an anvil rod and staple cartridge assembly of the surgical stapling apparatus of FIGS. 9A, 9B, and 10 within the intestinal area; and
FIG. 12 is a schematic perspective view of the intestinal area of FIG. 11, illustrating the anvil rod mounted to the surgical stapling apparatus.

### DETAILED DESCRIPTION OF THE EMBODIMENTS

Various exemplary embodiments of the present disclosure are discussed herein below in terms of buttresses for use with surgical stapling apparatus. The buttresses described herein may be used in sealing a wound by approximating the edges of wound tissue between a staple cartridge and an anvil plate of a surgical stapling apparatus which contains at least one surgical buttress. The at least one surgical buttress is joined to the surgical stapling apparatus by a strategically placed adhesive member, which can be an adhesive tape. The adhesive member is placed adjacent a central longitudinal slot and positioned between a tissue contacting surface of each of the staple cartridge and anvil plate and the least one surgical buttress. Firing of the surgical stapling apparatus forces legs of at least one staple to pass through an opening on the staple cartridge, the tissue, and the openings on the anvil plate to secure the surgical buttress to the tissue, to secure the adjoining tissue to one another. The buttress can help seal the tissue, and support the tissue. The firing force of the staple impacts the adhesive member to break the bond between the adhesive member and the surgical buttress thereby releasing the surgical buttress from the tissue contacting surface. As a knife blade translates distally within the central longitudinal slot, the adhesive tape is cut and fully releases the surgical buttress from the tissue contacting surface. Thus, the present disclosure describes surgical buttresses, surgical stapling apparatus supporting said surgical buttresses, and methods and mechanisms for using the same.

It should be understood that a variety of surgical stapling apparatus may be utilized with a surgical buttress of the present disclosure. For example, linear stapler configurations may be utilized, such as, for example those including Duet TRS™ reloads and staplers with Tri-Staple™ technology, available through Covidien, which maintain a principal place of business at 555 Long Wharf Drive, North Haven, CT 06511, and transverse anastomosis staplers, such as, for example, EEA™, CEEA™, GIA™, EndoGIA™, and TA™ staplers, also available through Covidien. It should also be appreciated that the principles of the present disclosure are equally applicable to surgical staplers having alternate configurations, such as, for example, end-to-end anastomosis staplers having a circular cartridge and anvil (see, e.g., commonly owned U.S. Patent No. 5,915,616, entitled "Surgical Fastener Applying Apparatus,"); laparoscopic staplers (see, e.g., commonly owned U.S. Pat. Nos. 6,330,965 and 6,241,139, each entitled "Surgical Stapling Apparatus,"); and transverse anastomosis staplers (see, e.g., commonly owned U.S. Patent Nos. 5,964,394 and 7,334,717, each entitled "Surgical Fastener Applying Apparatus").

Embodiments of the presently disclosed surgical buttress and surgical stapling apparatus will now be described in detail with reference to the drawing figures wherein like reference numerals identify similar or identical elements. In the following discussion, the terms "proximal" and "trailing" may be employed interchangeably, and should be understood as referring to the portion of a structure that is closer to a clinician during proper use. The terms "distal" and "leading" may also be employed interchangeably, and should be understood as referring to the portion of a structure that is further from the clinician during proper use. As used herein, the term "patient" should be understood as referring to a human subject or other animal, and the term "clinician" should be understood as referring to a doctor, nurse, or other care provider and may include support personnel.

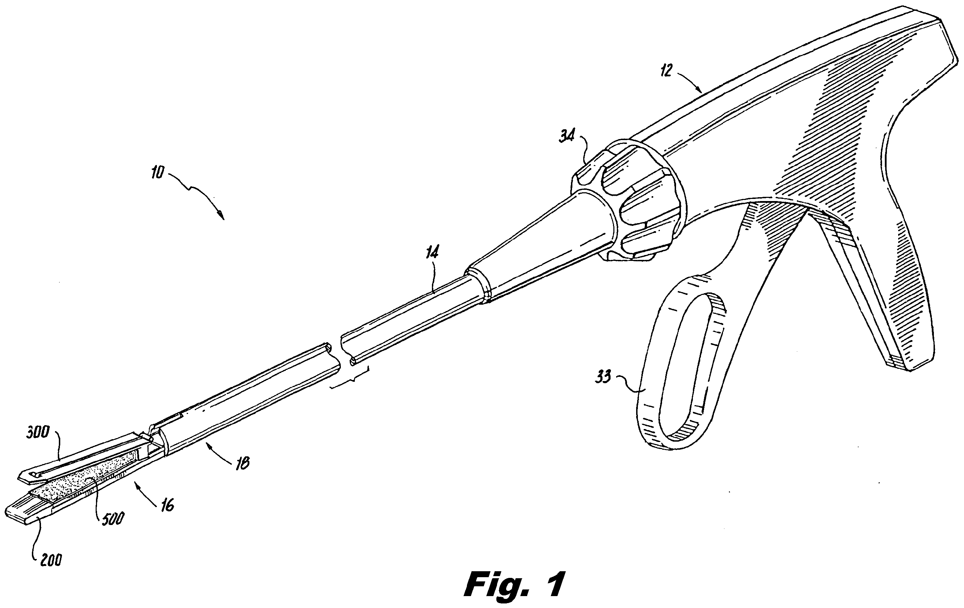

Referring now to FIG. 1, there is disclosed an exemplary surgical stapling apparatus or surgical stapler 10 for use in stapling tissue and applying a buttress material or surgical buttress to the tissue. A further example of this type of surgical stapling instrument is disclosed in U.S. Patent No. 7,128,253. The surgical stapling instrument can have a removable and replaceable loading unit that includes the cartridge assembly and anvil assembly, a surgical stapling instrument with a removable and replaceable cartridge assembly, or both. The surgical stapling instrument may have a manually driven handle assembly or a handle assembly that is powered by electrical motor, pressurized gas or other fluid, etc.

Surgical stapling apparatus 10 generally includes a handle 12 having an elongate tubular member 14 extending distally from handle 12. An end effector assembly 16 is mounted on a distal end 18 of elongate tubular member 14. End effector assembly 16 includes a first jaw or staple cartridge assembly 200 configured to receive a staple cartridge 32 therein and a second jaw or anvil assembly 300. End effector assembly 16 may be permanently affixed to elongate tubular member 14 or may be detachable and thus replaceable with a new end effector assembly 16. One of staple cartridge assembly 200 and anvil assembly 300 is movably mounted at distal end 18 of end effector assembly 16, and is movable between an open position spaced apart from one another to a closed position substantially adjacent to one another. Anvil assembly 300 supports an anvil plate 302 and is fabricated from a material appropriate to surgical uses, such as a metal material including and not limited to stainless steel, titanium, titanium alloy, and the like. At least a tissue contacting surface of staple cartridge 32 is fabricated from a material other than metal, including and not limited to plastic, thermoplastic, resin, polycarbonate, and the like.

Surgical stapling apparatus 10 further includes a trigger 33, as seen in FIG. 1, movably mounted on handle 12. Actuation of trigger 33 initially operates to move first jaw and second jaw between the open and the closed positions and simultaneously actuates surgical stapling apparatus 10 to apply lines of staples to tissue. In order to properly orient end effector assembly 16 relative to the tissue to be stapled, surgical stapling apparatus 10 is additionally provided with a rotation knob 34 mounted on handle 12. Rotation of rotation knob 34 relative to handle 12 rotates elongate tubular member 14 and end effector assembly 16 relative to handle 12 so as to properly orient end effector assembly 16 relative to the tissue to be stapled. The surgical stapling instrument may may or may not have jaws that can articulate or pivot with respect to the elongate member 14.

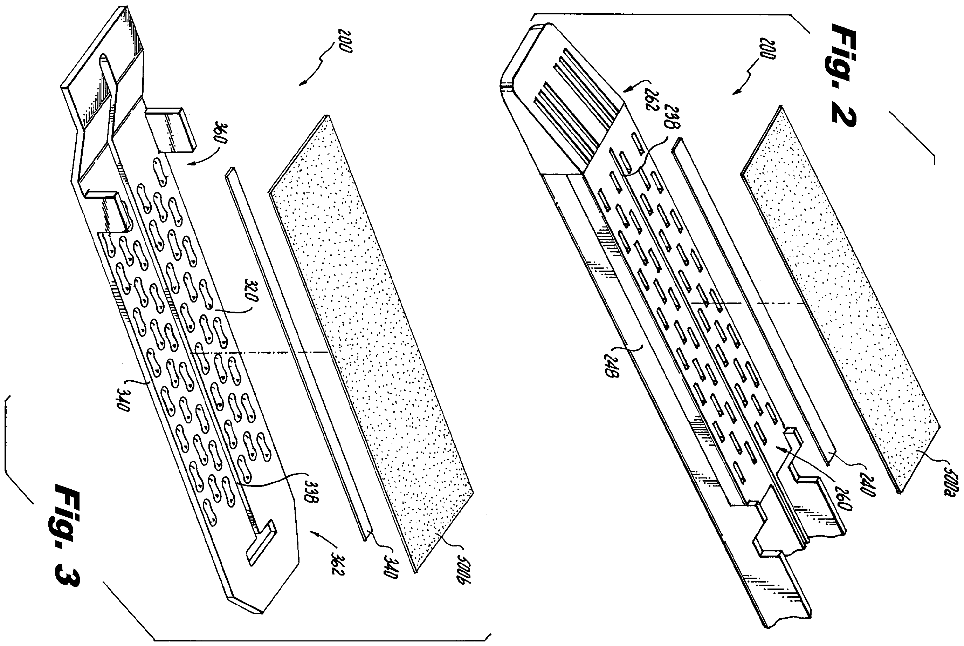

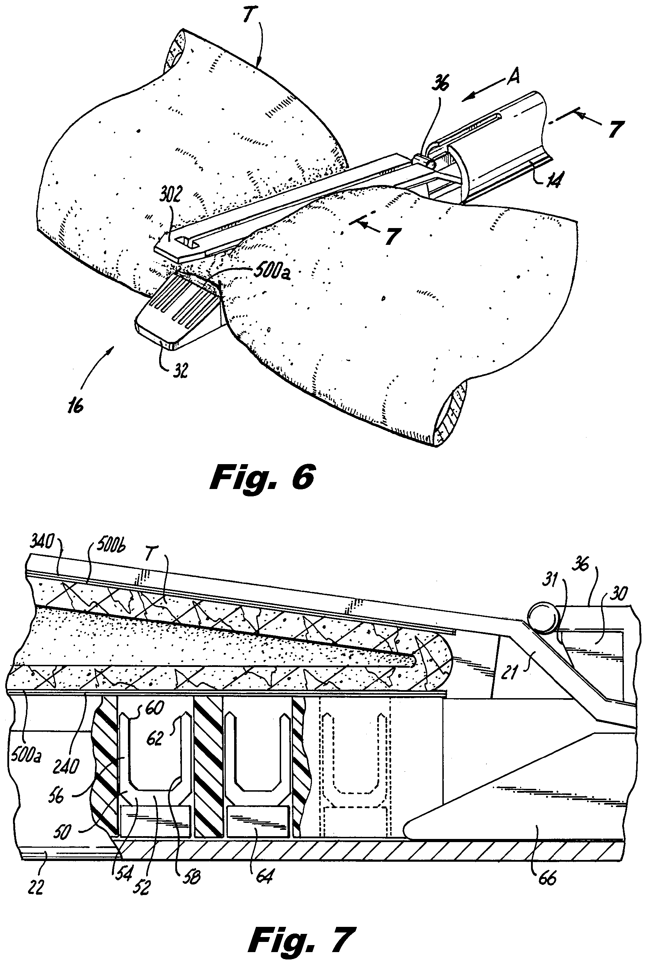

A driver 36, as seen in FIGS. 6 and 7A, is provided to move or approximate first jaw or staple cartridge assembly 200 and second jaw or anvil assembly 300 from the open position to the closed position. Driver 36 moves through a longitudinal slot 338 (FIG. 3) formed in the anvil plate 302 of anvil assembly 300. A knife 30 with knife blade 31 is associated with driver 36 to cut tissue captured between staple cartridge assembly 200 and anvil assembly 300 as driver 36 passes through slot 338. The driver desirably engages both the cartridge assembly and the anvil assembly to clamp tissue, and travels through a slot in the staple cartridge that corresponds to slot 338.

Reference may be made to commonly owned U.S. Patent Nos. 5,915,616, 6,330,965, and 6,241,139, referenced above, for a detailed discussion of the construction and operation of an exemplary surgical stapling apparatus 10.

Staple cartridge assembly 200 and/or anvil assembly 300 may be provided with a surgical buttress 500. Surgical buttress 500 is provided to reinforce and seal the lines of staples applied to tissue by surgical stapling apparatus 10. Surgical buttress 500 may be configured into any shape, size, or dimension suitable to fit any surgical stapling, fastening, or firing apparatus.

Staple cartridge assembly 200 is provided with a cartridge buttress 500a and anvil assembly 300 is provided with an anvil buttress 500b in the manners described in more detail hereinbelow. The buttresses 500a, 500b may be made from any biocompatible natural or synthetic material. The material from which the buttresses 500a, 500b are formed may be bioabsorbable or non-bioabsorbable. It should be understood that any combination of natural, synthetic, bioabsorbable and non-bioabsorbable materials may be used to form the buttress material. The buttresses 500a, 500b may be porous or non-porous, combination of porous and non-porous layers. The non-porous buttresses 500a, 500b may be utilized to retard or prevent tissue ingrowth from surrounding tissues thereby acting as an adhesion barrier and preventing the formation of unwanted scar tissue. For example, the buttress material can include polyglycolic acid, glycolide, trimethylene carbonate, animal derived materials such as porcine or bovine collagen, etc. The buttress material can be formed as a sheet that is molded or extruded, for example; non-woven materials, mesh materials, foams and the like are also contemplated.

Additional exemplary materials for surgical buttresses 500a, 500b for use with the surgical stapling devices disclosed herein are set forth in commonly assigned U.S. Patent Nos. 5,542,594; 5,908,427; 5,964,774; and 6,045,560, and commonly assigned U.S. Application Publication Nos. 2006/0085034, filed on April 20, 2006; and 2006/0135992, filed on June 22, 2006.

As illustrated in the current embodiment and shown in FIG. 2 and 3, surgical buttress 500 is releasably attached to staple cartridge assembly 200 and/or anvil assembly 300 by strategically positioned adhesive members or tapes 240, 340 that affix surgical buttresses 500a, 500b to the inwardly facing or tissue contacting surfaces 220, 320 of the staple cartridge 32 and/or the anvil plate 302, as discussed in detail below.

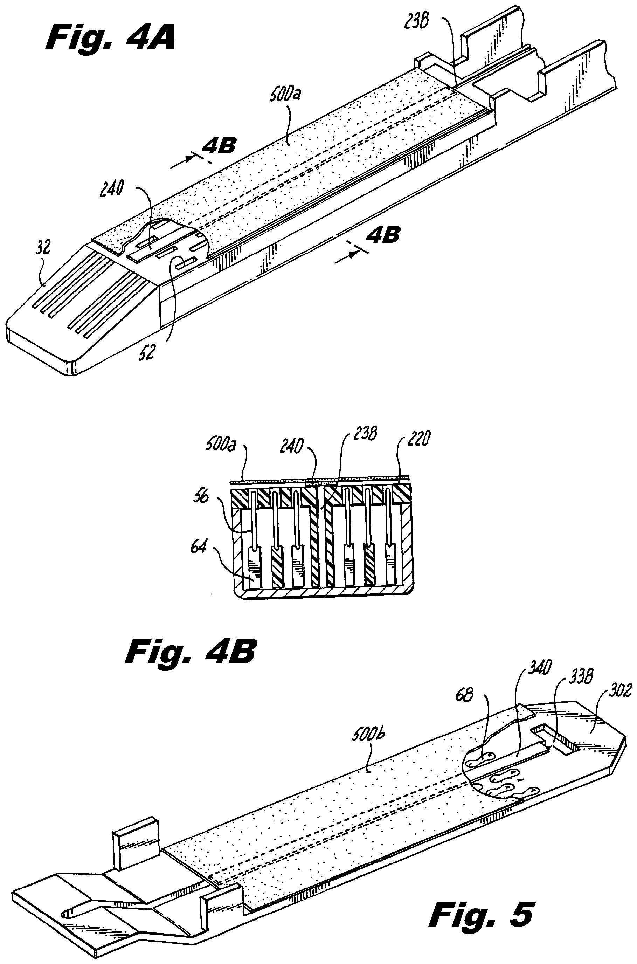

With reference to FIG. 2, cartridge buttress 500a of staple cartridge assembly 200 is operatively secured or adhered to a tissue contacting surface 220 of staple cartridge 32, by an adhesive tape 240 positioned adjacent to and over a central longitudinal slot 238 along the tissue contacting surface 220. Adhesive tape 240 is disposed between the cartridge buttress 500a and the tissue contacting surface 220. Adhesive tape 240 extends longitudinally from a proximal end 260 to a distal end 262 of staple cartridge 32. FIG. 2 illustrates adhesive tape 240 as one single piece, however, adhesive tape 240 may comprise a plurality of small pieces spaced longitudinally along tissue contacting surface 220. The length and width of adhesive tape 240 may vary depending on the staple cartridge 32. According to the invention the adhesive tape 240 does not extend over the staple retaining slots 52 of staple cartridge 32. More specifically, adhesive tape 240 is disposed on the tissue contacting surface 220 surrounding the edges 248 of the central longitudinal slot 238 (FIG. 4A) and does not cover the staple retaining slots 52 of staple cartridge 32. By allowing staple retaining slots 52 to remain uncovered by adhesive tape 240, staples 50 may penetrate tissue without any additional obstacles. Cartridge buttress 500a is preferably laser welded onto the adhesive tape 240. Other methods of binding or securing the cartridge buttress 500a to the adhesive tape 240 are contemplated such as ultrasonic welding, solvent bonding, or heat pressing.

With reference to FIG. 3, and similar to cartridge buttress 500a, anvil buttress 500b is operatively secured or adhered to a tissue contacting surface 320 of anvil plate 302 of anvil assembly 300 by an adhesive tape 340 surrounding the edges 348 of the central longitudinal slot 338 along the tissue contacting surface. Adhesive tape 340 extends from a proximal end 360 and a distal end 362. Preferably, adhesive 340 does not extend over the staple forming pockets 68 of anvil plate 302.

FIGS. 4A and 5 illustrate the buttresses 500a, 500b disposed on the staple cartridge 32 and anvil plate 302, respectively. As shown, adhesive tapes 240, 340 are disposed over the respective central longitudinal slots 238, 338 and between the tissue contacting surfaces 220, 320 and the buttresses 500a, 500b. FIG. 4B shows a cross-sectional view of the cartridge buttress 500a disposed on the staple cartridge 32 showing the adhesive tape 240 disposed between the cartridge buttress 500a and the tissue contacting surface 220. Further adhesive tape 240 is disposed over and surrounding the edges 248 of the central longitudinal slot 238. While FIG. 4B is directed to the staple cartridge it is understood that a similar construction is utilized for the anvil plate. In accordance with the present disclosure, adhesive tapes 240, 340 do not extend over the staple forming recesses 68 of anvil plate 302 or over the staple retaining slots 52 of the staple cartridge 32, respectively.

The adhesive tapes 240, 340 may be of varying widths and thickness depending upon the staple cartridge 32 and anvil plate 302. Preferably, the adhesive tapes 240, 340 are 2.18mm (0.086 inches) in width and between 0.0508-0.1016mm (.002-.004 inches) in thickness. Generally, the central longitudinal slots 238, 338 are 1.27mm (0.050 inches) in width. Therefore, the adhesive tapes 240, 340 extend 0.457mm (0.018 inches) over each of the edges 248, 348 of the respective central longitudinal slots 238, 338 of each of the staple cartridge 32 and anvil plate 302. However, other shapes and sizes are contemplated, the configuration of the adhesive member or adhesive tape being dependent on the type of stapler.

During assembly adhesive tapes 240, 340 are placed onto each of the tissue contacting surfaces 220, 320 of staple cartridge assembly 200 and anvil assembly 300, respectively. Buttresses 500a, 500b are next placed atop the adhesive tapes 240, 340 and laser welded thereto.

As illustrated in FIG. 6, during use of surgical stapling apparatus 10, the first jaw or staple cartridge assembly 200 and the second jaw or anvil assembly 300, having surgical buttresses 500a, 500b loaded thereon (as described above) are positioned on either side of the surgical site. Tissue contacting surfaces 220, 320 of staple cartridge assembly 200 and anvil assembly 300 are positioned adjacent layers of tissue "T" to be fastened to one another.

As shown in FIG. 7, staple cartridge assembly 200 includes surgical staples 50 positioned within individual staple retaining slots 52 of staple cartridge 32. Staples 50 are of a conventional type and include a backspan 54 having a pair of legs 56 and 58 extending from backspan 54. Legs 56 and 58 terminate in tissue penetrating tips 60 and 62, respectively. Pushers 64 are located within staple retaining slots 52 and are positioned between staples 50 and the path of a drive bar 66.

In the illustrated embodiment, surgical stapling apparatus 10 is initially actuated by movement of trigger 33 relative to handle 12 (FIG. 1) causing driver 36 to move in the direction of arrow "A" (FIG. 6), and against sloped edge 21 of anvil plate 302 thereby causing anvil assembly 300 to be moved to the closed position relative to staple cartridge assembly 200. As drive bar 66 advances distally within staple cartridge 32, drive bar 66 urges pushers 64 upwardly against backspan 54 of staples 50 driving legs 56 and 58 of staples 50 through the cartridge buttresses 500a, tissue "T", and anvil buttress 500b, towards staple forming pockets 68 in anvil plate 302 of anvil assembly 300. Tissue penetrating tips 60 and 62 of staple legs 56 and 58 are bent within staple forming pockets 68 in anvil plate 302 with backspan 54 securing surgical buttress 500 against tissue "T". The firing force of the surgical stapling apparatus 10 begins to break the bond between the buttresses 500a, 500b and adhesive tapes 240, 340.

In alternate embodiments, if adhesive tapes 240, 340 are fabricated from bio-absorbable materials, it is contemplated that the firing force of the surgical stapling apparatus 10 begins to break the bond between the buttresses 500a, 500b and respective tissue contacting surfaces of staple cartridge 32 and anvil plate 302.

Opening end effector assembly 16, after firing, releases the bond between the buttresses 500a, 500b and adhesive tapes 240, 340 in order to release the buttresses 500a, 500b from the respective tissue contacting surfaces 220, 320 of the staple cartridge 32 and anvil plate 302. Upon full actuation of surgical stapling apparatus 10, a knife 30 (FIG. 7) associated with surgical stapling apparatus 10 and carried by driver 36 is utilized to cut tissue "T", as well as surgical buttresses 500a, 500b and adhesive tapes 240, 340 between the rows of now formed staples 50. Upon movement of anvil assembly 300 to the open position, spaced apart from staple cartridge assembly 200, buttresses 500a, 500b are pulled away from the adhesive tapes 240, 340, respective tissue contacting surfaces 220, 320 of respective staple cartridge assembly 200 and anvil assembly 300.

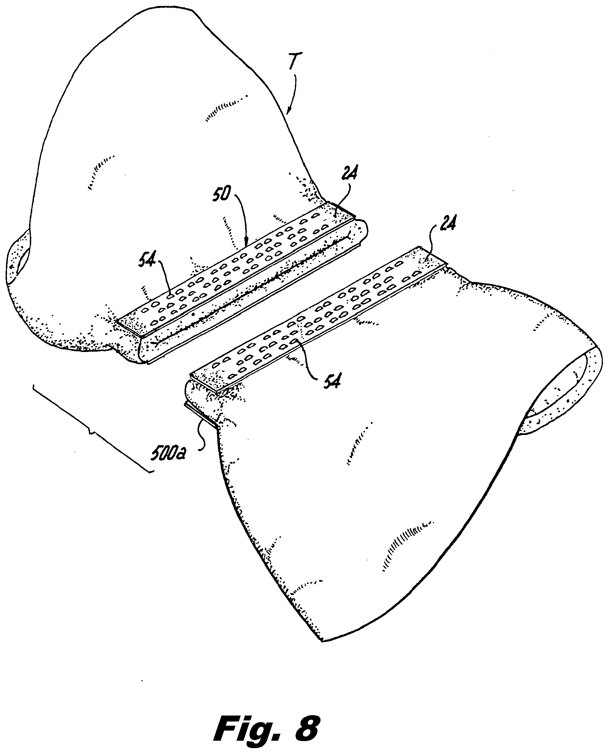

The resulting tissue "T", divided and stapled closed with staples 50, is illustrated in FIG. 8. Specifically, surgical buttresses 500a, 500b are secured against tissue "T" by legs 56, 58 and backspans 54 of staples 50. Thus, surgical buttresses 500a, 500b are stapled to tissue "T" thereby sealing and reinforcing the staple lines created by staples 50.

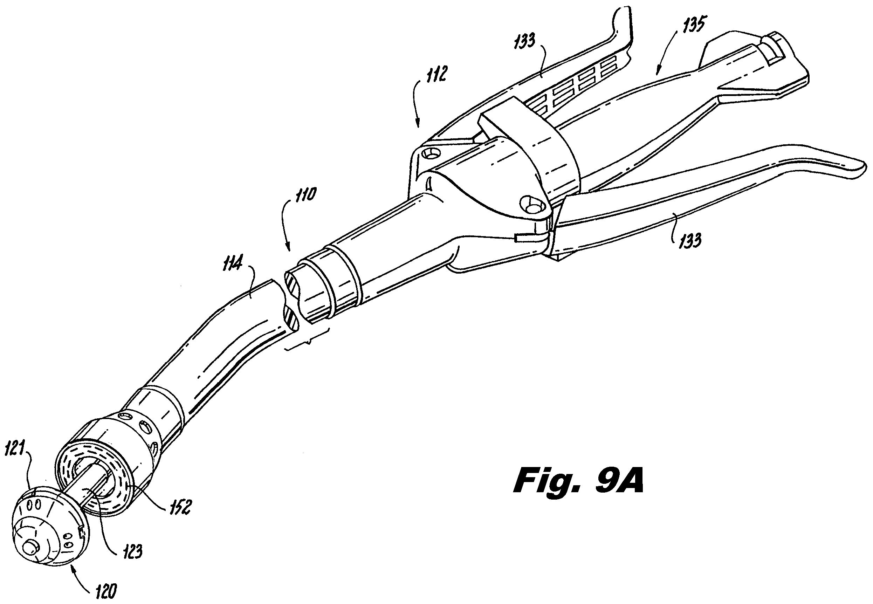

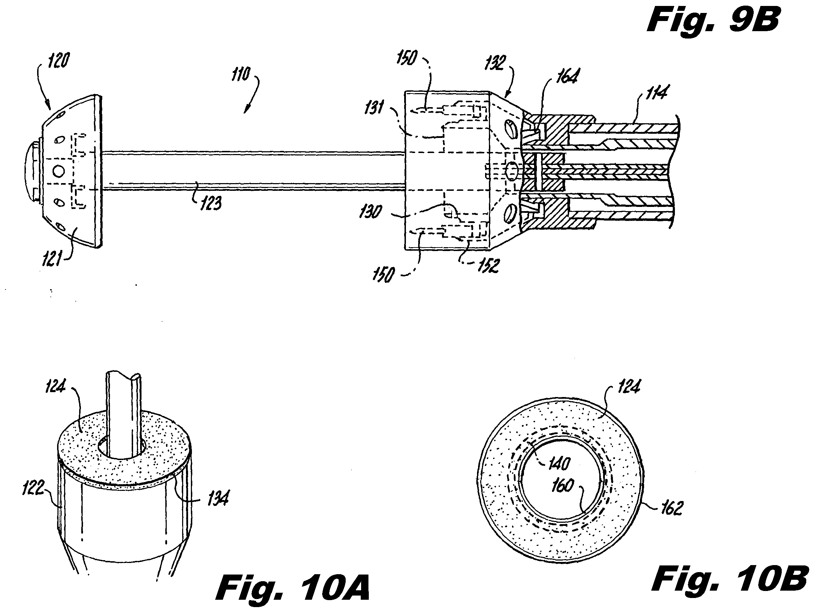

Referring now to FIGS. 9A and 9B, an annular surgical stapling apparatus 110, for use with surgical buttresses 124 of the present disclosure, is shown, not forming part of the invention as claimed. Surgical stapling apparatus 110 includes a handle assembly 112 having at least one pivotable actuating handle member 133, and an advancing member 135. Extending from handle member 112, there is provided a tubular body portion 114 which may be constructed so as to have a curved shape along its length. Body portion 114 terminates in a staple cartridge assembly 122 which includes a pair of annular arrays of staple retaining slots 152 having a staple 150 disposed in each one of staple retaining slots 152. Positioned distally of staple cartridge 122 there is provided an anvil assembly 120 including an anvil member 121 and a shaft 123 operatively associated therewith for removably connecting anvil assembly 120 to a distal end portion of stapling apparatus 110.

Staple cartridge assembly 122 may be fixedly connected to the distal end of tubular body portion 114 or may be configured to concentrically fit within the distal end of tubular body portion 114. Staple cartridge assembly 122 includes a staple pusher 164 including a proximal portion having a generally frusto-conical shape and a distal portion defining two concentric rings of peripherally spaced fingers (not shown), each one of which is received within a respective staple retaining slot 152.

A knife 130, substantially in the form of an open cup with the rim thereof defining a knife blade 131, is disposed within staple cartridge assembly 122 and mounted to a distal surface of a staple pusher 164. The knife 130 is disposed radially inward of the pair of annular arrays of staples 150. Accordingly, in use, as the staple pusher 164 is advanced, the knife 130 is also advanced axially distally.

As seen in FIG. 10A, a surgical buttress 124 is releasably attached to the staple cartridge assembly 122 an adhesive tape 140 disposed between the surgical buttress 124 and the tissue contacting surface 134 of the staple cartridge assembly 122. As described herein above, adhesive tape 140 bonds the surgical buttress 124 to the tissue contacting surface 134. Surgical buttress 124 is provided in an annular configuration and includes a central aperture 125 to receive shaft 123 of anvil assembly 120 therethrough.

It is envisioned that the surgical buttress 124 may be additionally or alternatively attached or adhered to tissue contacting surface of anvil plate 121 in a manner similar to the surgical buttress 124 attached to staple cartridge assembly 122.

As shown in FIG. 10B, surgical buttress 124 may be secured or adhered to the staple cartridge 122 along an inner portion 160. It is envisioned that other configurations may be utilized to retain the surgical buttress 124 to the staple cartridge assembly 122, such as placing of adhesive tape 140 along the outer portion 162, or alternating a plurality of pieces of adhesive tapes 140 between the inner portion 160 and outer portion 162, or among other arrangements within the purview of those skilled in the art.

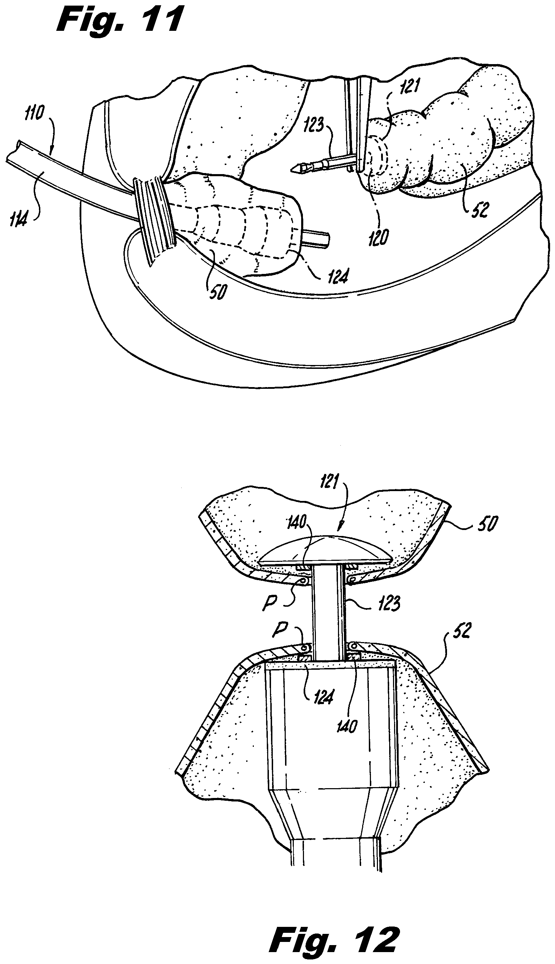

Surgical stapling apparatus 110 and detachable anvil assembly 120 are used in an anastomosis procedure to effect joining of intestinal sections 50 and 52. The anastomosis procedure is typically performed using minimally invasive surgical techniques including laparoscopic means and instrumentation. At the point in the procedure shown in FIG. 11, a diseased intestinal section has been previously removed, anvil assembly 120 (optionally including a surgical buttress 124 thereon) has been applied to the operative site either through a surgical incision or transanally and positioned within intestinal section 52, and tubular body portion 114 of surgical stapling apparatus 110 (optionally including a surgical buttress 124 thereon) has been inserted transanally into intestinal section 50. Intestinal sections 50 and 52 are also shown temporarily secured about their respective components (e.g., shaft 123 of anvil assembly 120, and the distal end of tubular body portion 114) by conventional means such as a purse string suture "P", as illustrated in FIG. 12.

Thereafter, the clinician maneuvers anvil assembly 120 until the proximal end of shaft 123 is inserted into the distal end of tubular body portion 114 of surgical stapling apparatus 110, wherein the mounting structure (not shown) within the distal end of tubular body portion 114 engages shaft 123 to effect the mounting. Anvil assembly 120 and tubular body portion 114 are then approximated to approximate intestinal sections 50, 52. Surgical stapling apparatus 110 is then fired. A knife (not shown) cuts the portion of tissue and surgical buttress 124 disposed radially inward of the knife, to complete the anastomosis. The force of the opening of anvil assembly 120 and staple cartridge assembly 122, with surgical buttress 124 stapled to intestinal sections 50 and 52, causes surgical buttress 124 to release at the attachment pads 140 thereby releasing the surgical buttress 124 from the tissue contacting surface 134.

Persons skilled in the art will understand that the devices and methods specifically described herein and illustrated in the accompanying figures are non-limiting exemplary embodiments, and that the description, disclosure, and figures should be construed merely exemplary of particular embodiments. It is to be understood, therefore, that the present disclosure is not limited to the precise embodiments described, and that various other changes and modifications may be effected by one skilled in the art without departing from the scope of the disclosure. Additionally, it is envisioned that the elements and features illustrated or described in connection with one exemplary embodiment may be combined with the elements and features of another exemplary embodiment without departing from the scope of the present disclosure, and that such modifications and variations are also intended to be included within the scope of the present disclosure. Accordingly, the subject matter of the present disclosure is not to be limited by what has been particularly shown and described, except as indicated by the appended claims.

## Claims

1. A staple cartridge (32) for use with a surgical stapling apparatus, the staple cartridge (32) comprising: a cartridge body including a tissue contacting surface (220) defining a plurality of staple retaining slots (52) and having a central longitudinal slot (238) having edges; a surgical buttress (500a) releasably disposed on the tissue contacting surface (220) of the cartridge body; and an adhesive member disposed over the central longitudinal slot (238) of the cartridge body and configured to retain the surgical buttress (500a) atop the tissue contacting surface (220) of the cartridge body, wherein the adhesive member is an adhesive tape (240) which is secured to the tissue contacting surface (220) of each of the staple cartridge (32) surrounding the edges of the central longitudinal slot (238), wherein the surgical buttress (500a) is secured to the adhesive tape (240);
the staple cartridge being **characterized in that**: the adhesive tape (240) does not extend over the staple retaining slots (52).

2. The staple cartridge (32) of claim 1, wherein the surgical buttress (500a) is secured to the adhesive tape (240) by laser welding.

3. The staple cartridge (32) of claim 1 or claim 2, wherein the adhesive tape (240) extends longitudinally between a proximal end and a distal end of the tissue contacting surface (220) of the cartridge body.

4. An end effector (16) for use with a surgical apparatus, the end effector (16) comprising: a staple cartridge (32) according to any of claims 1 to 3; an anvil plate (302) having a tissue contacting surface (320) defining plurality of staple forming pockets (68) and having a central longitudinal slot (338) having edges; a surgical buttress (500b) releasably disposed on the tissue contacting surface (330) of the anvil plate (302); and an adhesive member disposed over the central longitudinal slot (338) of the anvil plate (302) and configured to retain the respective surgical buttress (500b) atop the anvil tissue contacting surface (320), wherein the adhesive member is an adhesive tape (340) secured to the tissue contacting surface (320) of each of the anvil plate (302) surrounding the edges of the anvil central longitudinal slot (338), wherein the surgical buttress (500b) is secured to the adhesive tape (340),
**characterised in that** the adhesive tape (340) does not extend over the staple forming pockets (68).

## Patentansprüche

1. Klammerkassette (32) zur Verwendung mit einem chirurgischen Heftgerät, wobei die Klammerkassette (32) umfasst: einen Kassettenkörper mit einer Gewebekontaktfläche (220), die mehrere Klammerhalteschlitze (52) definiert und einen zentralen Längsschlitz (238) mit Kanten aufweist; eine chirurgische Versteifung (500a), die lösbar auf der Gewebekontaktfläche (220) des Kassettenkörpers angeordnet ist; und ein Klebeelement, das über dem zentralen Längsschlitz (238) des Kassettenkörpers angeordnet und konfiguriert ist, um die chirurgische Versteifung (500a) auf der Gewebekontaktfläche (220) des Kassettenkörpers zu halten, wobei das Klebeelement ein Klebeband (240) ist, das an der Gewebekontaktfläche (220) jeder der die Ränder des zentralen Längsschlitzes (238) umgebenden Klammerkassetten (32) befestigt ist, wobei die chirurgische Versteifung (500a) an dem Klebeband (240) befestigt ist;
wobei das Klammermagazin **dadurch gekennzeichnet ist, dass** sich das Klebeband (240) nicht über die Klammerhalteschlitze (52) erstreckt.

2. Klammerkassette (32) nach Anspruch 1, wobei das chirurgische Sägezahngewinde (500A) auf das Klebeband (240) durch Laserschweißen befestigt ist.

3. Klammerkassette (32) nach Anspruch 1 oder Anspruch 2, wobei sich das Klebeband (240) in Längsrichtung zwischen einem proximalen Ende und einem distalen Ende der Gewebekontaktfläche (220) des Kassettenkörpers erstreckt.

4. Endeffektor (16) zur Verwendung mit einer chirurgischen Vorrichtung, wobei der Endeffektor (16) Folgendes umfasst:
ein Klammermagazin (32) nach einem der Ansprüche 1 bis 3; eine Ambossplatte (302) mit einer Gewebekontaktfläche (320), die mehrere Taschen (68) zur Bildung von Klammern definiert und
einen zentralen Längsschlitz (338) mit Kanten aufweist; eine chirurgische Versteifung (500b), die lösbar auf der Gewebekontaktfläche (330) der Ambossplatte (302) angeordnet ist; und ein Klebeelement, das über dem zentralen Längsschlitz (338) der Ambossplatte (302) angeordnet und konfiguriert ist, um die jeweilige chirurgische Versteifung (500b) auf der Ambossgewebekontaktfläche (320) zu halten, wobei das Klebeelement ein Klebeband ist (340), das an einer Gewebekontaktfläche (320) jeder Ambossplatte (302) befestigt ist, welche die Kanten des zentralen Längsschlitzes (338) des Ambosses umgibt, wobei die chirurgische Versteifung (500b) an dem Klebeband (340) befestigt ist, **dadurch gekennzeichnet, dass** sich das Klebeband (340) nicht über die die Klammern bildenden Taschen (68) erstreckt.

## Revendications

1. Cartouche d'agrafes (32) destinée à être utilisée avec un appareil d'agrafage chirurgical, la cartouche d'agrafes (32) comprenant : un corps de cartouche comportant une surface de contact tissulaire (220) définissant une pluralité de fentes de retenue d'agrafes (52) et présentant une fente longitudinale centrale (238) présentant des bords ; un renfort chirurgical (500a) disposé de manière libérable sur la surface de contact tissulaire (220) du corps de cartouche ; et un élément adhésif disposé par-dessus la fente longitudinale centrale (238) du corps de cartouche et conçu pour retenir le renfort chirurgical (500a) sur la surface de contact tissulaire (220) du corps de cartouche, l'élément adhésif étant un ruban adhésif (240) qui est fixé à la surface de contact tissulaire (220) de la cartouche d'agrafes (32) entourant les bords de la fente longitudinale centrale (238), le renfort chirurgical (500a) étant fixé au ruban adhésif (240) ;
la cartouche d'agrafes étant **caractérisée en ce que** : le ruban adhésif (240) ne s'étend pas par-dessus les fentes de retenue d'agrafes (52).

2. Cartouche d'agrafes (32) selon la revendication 1, dans laquelle le renfort chirurgical (500a) est fixé au ruban adhésif (240) par soudage au laser.

3. Cartouche d'agrafes (32) selon la revendication 1 ou la revendication 2, dans laquelle le ruban adhésif (240) s'étend longitudinalement entre une extrémité proximale et une extrémité distale de la surface de contact tissulaire (220) du corps de cartouche.

4. Effecteur terminal (16) destiné à être utilisé avec un appareil chirurgical, l'effecteur terminal (16) comprenant :
une cartouche d'agrafes (32) selon l'une quelconque des revendications 1 à 3 ; une plaque d'enclume (302) présentant une surface de contact tissulaire (320) définissant une pluralité de poches de formation d'agrafes (68) et présentant une fente longitudinale centrale (338) présentant des bords ; un renfort chirurgical (500b) disposé de manière libérable sur la surface de contact tissulaire (330) de la plaque d'enclume (302) ; et un élément adhésif disposé par-dessus la fente longitudinale centrale (338) de la plaque d'enclume (302) et conçu pour retenir le renfort chirurgical respectif (500b) sur la surface de contact tissulaire (320) de l'enclume, l'élément adhésif étant un ruban adhésif (340) fixé à la surface de contact tissulaire (320) de la plaque d'enclume (302) entourant les bords de la fente longitudinale centrale (338) de l'enclume, le renfort chirurgical (500b) étant fixé au ruban adhésif (340), **caractérisé en ce que** le ruban adhésif (340) ne s'étend pas par-dessus les poches de formation d'agrafes (68).
